(19) Europäisches Patentamt
European Patent Office
Office européen des brevets

(11) Publication number: 0 336 417 A2

(12) # EUROPEAN PATENT APPLICATION

(21) Application number: 89106066.7

(22) Date of filing: 06.04.89

(51) Int. Cl.4: C08F 2/50 , C08F 4/00 , A61K 6/00

(30) Priority: 07.04.88 US 178679

(43) Date of publication of application: 11.10.89 Bulletin 89/41

(84) Designated Contracting States: DE ES FR GB NL SE

(71) Applicant: DENTSPLY INTERNATIONAL, INC.
570 West College Avenue P.O. Box 872
York Pennsylvania 17405(US)

(72) Inventor: Venz, Sabine, Dr.
19223 Dunbridge Way
Gaithersburg, MD 20879(US)

(74) Representative: Wächtershäuser, Günter, Dr.
Tal 29
D-8000 München 2(DE)

(54) **Phosphonate initiators for light cured compositions.**

(57) An initiator system for photo curing olefinically unsaturated monomers is provided. The initiator system, in its preferred embodiment comprises an acylphosphonate, camphorquinone and an amine. Using the initiator of the invention produces a cured polymer with unexpectedly improved properties.

EP 0 336 417 A2

Xerox Copy Centre

# PHOSPHONATE INITIATORS FOR LIGHT CURED COMPOSITIONS

## BACKGROUND OF THE INVENTION

This invention relates to initiators used with dental compositions which are curable by free radical polymerization and, preferably, by the action of visible light. More particularly, use of the initiators with one-component dental composite formulations and urethane impression material formulations are disclosed.

Dental restoratives should exhibit certain obligatory physical and chemical characteristics in order to be suitable for use in filling, repairing or replacing teeth. Thus, restorative materials should possess properties that closely match natural teeth with respect to structural properties such as cohesive strength, coefficient of thermal expansion and wearability. Also, aesthetic considerations such as color stability, refractive index, plaque repellency, polishability and opacity are important factors in determining whether a material is suitable for use as a dental restorative. In the past, numerous compositions have been tried in various mixtures and proportions in order to find satisfactory materials for use as dental composites or restoratives. These compositions have usually included some type of resin, which may either be preblended or mixed by the practitioner in the office, together with other materials such as pigments, catalysts, handling agents and opacifiers. For restorative use, materials generally are "filled", that is, they contain inorganic, or in some cases, organic particulate material. Unfilled, these same resins are used as coatings, especially pit and fissure sealants in preventative dentistry procedures.

Examples of such polymerizable resins include those described in copending U.S. application 040,636 filed April 21, 1987 and used as described in U.S. Patent 4,514,174 and similar resins as known in the art cited and described in those documents. These resins may be used for producing rigid or elastomeric materials having a wide variety of use in dentistry.

Examples are urethane diacrylates which are used in dental impression materials, dental filling materials, pit and fissure sealers, bridge materials, denture construction, drug delivery systems, cements, liners, bases and intraoral bandages. Essentially such compositions include a free radical polymerizable resin and contain polymerization initiators, accelerators and optionally fillers and stabilizers.

It will be appreciated by those skilled in the art that the use of one-component photoactivated materials ready to use without mixing, are to be preferred over more traditional thermochemical catalyst or redox activated compositions because of the increased work time allowed by their use of photoinitiated polymerization. In a two-component catalyst or redox system, work time is determined by the reaction time once the catalyst is added to the resin component. In a one-component photocured system, the practitioner may take whatever time is necessary for forming or molding a dental impression or restoration into the tooth formation, and then effect extremely rapid curing by exposing the photocurable material to the appropriate wavelength of electromagnetic radiation. Moreover, with a one-component photocured system, the practitioner need not be concerned with properly measuring and mixing two-components to insure that the resultant cured compositions achieve the proper physical characteristics.

There are prior art dental materials which utilize photoinitiators that are sensitive to visible light having wavelengths from about 3,600 angstroms to about 6,000 angstroms. These materials generally would be preferred over those materials which are cured with ultraviolet radiation because visible light is attenuated to a lesser degree by tooth structure and the filler than is ultraviolet radiation. However, many previous attempts to develop dental formulations using visible light curing formulations have resulted in less than most desirable performance.

Accordingly, it is a principal object of this invention to provide light cured compositions which are useful for dental applications.

A further object is to provide dental compositions which provide products of high strength, exhibit a rapid cure time, but which exhibit good workability prior to curing.

Still other objects and advantages of the present invention will become apparent from the following description of the invention.

## SUMMARY OF THE INVENTION

In accordance with the invention an initiator of the following formula, suitable for use with dental composites, is provided:

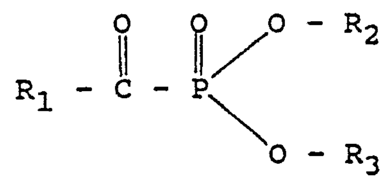

wherein $R_1$ is aryl, alkyl, OH or OR
wherein R is aryl or alkyl;
$R_2$ and $R_3$ can be the same or different and are selected from the group comprising aryl, alkyl and H. $R_2$ and $R_3$ together may form a ring. In the above formula alkyl may be a saturated or unsaturated aklyl group having from 1-18 carbon atoms, preferably 1-12 carbon atoms and most preferably 2-6 carbon atoms. Aryl represents an aromatic or heterocyclic ring or rings having 4-10 carbon atoms which may have substituents comprising halogen and lower alkyl comprising from 1-6 additional carbon atoms.

In accordance with the preferred embodiment of the invention, a photopolymerizable composition comprising an olefinically unsaturated monomer and a catalyst comprising a benzoyl di (2,6-dimethylphenyl) phosphonate is provided. The composition preferably includes at least one acrylic or methacrylic ester and may include an amine.

Also provided is a method of use of the above compositions for treating teeth by its application to a prepared tooth.

It has been found that the initiators of the invention provide a quicker, deeper cure than initiators used in the prior art.

## DETAILED DESCRIPTION OF THE INVENTION

In general, the properties of a resin used for dental applications, especially as a component of a composite, are improved when there is high conversion of monomer (i.e. the monomer substantially is completely cross-linked into the resulting polymer).

In some prior art light cured compositions, although a very good conversion of monomer can be obtained at the surface, the percentage of conversion of monomer decreases at greater depths in the resin or composite materials. (The percentage of conversion is measured spectrographically by quantifying the strength of spectrographic peaks corresponding to carbon-carbon unsaturation in the resin.) It has been theorized and demonstrated that the percentage of conversion decreases at greater depths in the resin because of a combination of the dispersion of light at greater depths, refraction, and the presence of oxygen which is a free radical scavenger which captures free radicals and short circuits or inhibits the free radical chain reaction initiated by the light source.

It is believed that the acylphosphonate initiators of the present invention achieve a better cure as evidenced by harder cured surfaces and a higher percentage of conversion at greater depths because the initiators can also act as an oxygen scavenger. That is, they remove oxygen from the radical reaction process which thereby extends the life of the free radical reaction initiated by the light source since less oxygen is available to scavange free radicals.

In accordance with the invention an initiator of the following formula, suitable for use with dental composites, is provided:

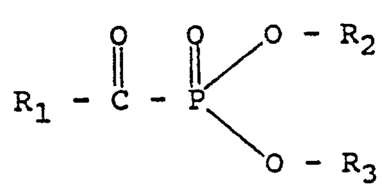

wherein $R_1$ is aryl, alkyl, OH or OR
wherein R is aryl or alkyl;
$R_2$ and $R_3$ can be the same or different and are selected from the group comprising aryl, alkyl and H. $R_2$ and $R_3$ together may form a ring. In the above formula alkyl may be a saturated or unsaturated alkyl group having from 1-18 carbon atoms, preferably 1-12 carbon atoms and most preferably 2-6 carbon atoms. Aryl

represents an aromatic or heterocyclic ring or rings having 4-10 carbon atoms which may have substituents comprising halogen and lower alkyl comprising from 1-6 additional carbon atoms.

When unsaturated, the alkyl group may comprise a polymerizable moiety and in a preferred embodi ment will comprise a methacrylate or acrylate group.

Benzoyl di-(2,6-dimethylphenyl) phosphonate is an example of a preferred acylphosphonate of the invention. Benzoyl di-(2,6-dimethylphenyl) phosphonate is available from BASF under the product name Lucerin 8728. The compound has maximum absorbance near 250 nm with a secondary absorbance peak in the near visible region at 380 nm. As is known to those skilled in the art, it is desirable to cure photocurable dental compositions in the near visible or visible wavelength range at about 360-620 nm, and preferably about 360-550 nm and most preferably 360-520 nm when cured in situ in the patient. Compositions using the present initiator may be cured at wavelengths of 250-620 nm when cured outside the patient.

Benzoyl di-(2,6-dimethylphenyl) phosphonate has the structure

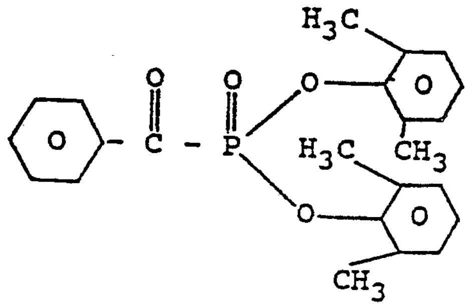

It has been found that benzoyl di (2,6 dimethylphenyl) phosphonate is a powerful photoinitiator which yields considerably higher percentage of cure than the best photoinitiator known in the prior art for dental compositions. It has been found that benzoyl di (2,6 dimethylphenyl) phosphonate can be mixed with NCO/TEGDMA in a concentration up to about 2 % without detrimental effect. NCO/TEGDMA is a resin blend described in copending SN 040, 636 and used as illustrated in U.S. patent 4,514,174 and comprises a blend of NCO, the reaction product of hexamethylene diisocyanate (HMDI) and Bis-GMA

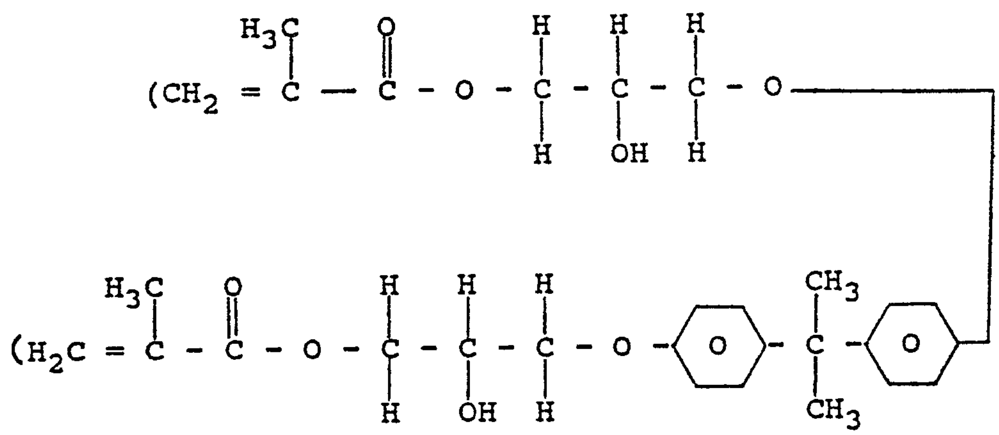

, and triethyleneglycol dimethacrylate (TEGDMA). At concentrations above about 2%, the benzoyl di (2,6 dimethylphenyl) phosphonate is not soluble in NCO/TEGDMA resin. See Table 1. When used with an NCO/TEGDMA resin in concentrations up to about 2%, however, the resin can be cured to a clear, hard film. Surprisingly, it has been found that benzoyl di (2,6 dimethylphenyl) phosphonate works well as an initiator alone, but does not have a great curing depth, but when used in combination with camphorquinone and an amine, the curing depth and degree of conversion are synergistically improved over a comparable system using only camphorquinone as an initiator and an amine as an accelerator.

As used herein, an amine used in the composition may be any amine known in the art for use as an accelerator in photocurable compositions. Preferably such an amine will have from 1-3 substituents, and will preferably be a tertiary amine, where in the substituents are selected from lower alkyl, acyl, aryl, ester or lower alkyl ester groups.

As used herein, lower alkyl may be a saturated or unsaturated carbon chain having 1-12 carbon atoms, aryl may have 6-12 carbon atoms and acyl and ester may have 1-12 carbon atoms and may include aryl substituents.

As used herein, "heterocyclic" group denotes a 5 to 7 membered aromatic or non-aromatic ring having carbon and 1-4 heteroatoms selected from N, O, S, Si, Se and mixtures thereof. More preferred are rings containing carbon and N, O, S or mixtures thereof.

The higher degree of cure of resins and composites containing benzoyl di (2,6 dimethylphenyl) phosphonate leads to a higher Knoop hardness, improved transverse strength and better resistance to solvents with varying solubility parameters. Compositions using benzoyl di (2,6 dimethylphenyl) phosphonate demonstrate a superior surface cure which improves a composite resin filling in a property that is desirable.

These properties are illustrated in the following tables:

Table I illustrates the improved conversion or crosslinking of monomer in the final polymer due to the use of Lucerin 8728 in the composition. In the table, Standard-M is a 50:50 NCO/TEGDMA resin blend without filler using camphorquinone initiator with methyldiethanolamine as an accelerator. SV-3-71-2 is standard M with the addition of 1% Lucerin 8728. It is noted that the presence of Lucerin 8728 improves the conversion of the resin by about 20% over the same composition without Lucerin 8728 (74% conversion versus 62% conversion). Similarly, standard E and standard Q are 50:50 NCO/TEGDMA resin blends using camphorquinone as an initiator and Ethyl 4 (N,N-dimethylaminobenzoate)

<u>i.e.</u> 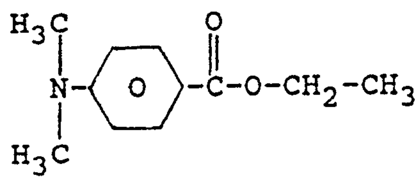

and Quantacure®(Ward and Blenkinsop) DMB (dimethylaminoethyl benzoate) <u>i.e.</u>

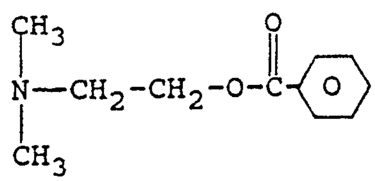:

respectively as accelerators.

Percentages are percentages by weight. Test data for these compositions illustrate improved conversion of monomer, regardless of the particular amine accelerator used, when using Lucerin, and Lucerin and camphorquinone together as an initiator system. Table I also illustrates that Lucerin alone is more effective than camphoquinone alone or camphorquinone plus an amine in the percentage of conversion (percentage of polymer cured). Further, it shows that Lucerin plus camphorquinone (no amine) is also effective. Accordingly, an amine is not required in an initiator system using Lucerin which contrasts with most prior art, especially camphorquinone initiator systems.

The degree of conversion was determined on thin resin films using FTIR spectroscopy. The films were prepared between Mylar sheets, irradiated for l0 seconds with the Prismetics Light and removed from the mylar after 15 minutes and stored in a dry oven for 24 hours between the irradiation and the IR-measurements to allow the completion of post curing.

In the calculation of the proportion of double bonds converted, the urethane absorption at about 3350 wavenumbers was used as an internal standard to calibrate the reduction of the vinyl absorption at 1637 wavenumbers.

TABLE I

| Formulations of Resins and Conversion of Double Bonds | | | | | | |
|---|---|---|---|---|---|---|
| Formulation | Resin | CQ | Lucerin 8728 | Amine | Conversion | Comment |
| Standard-M | NCO/TEGDMA | .15% | - | .50% MDEA | 62 % | |
| SV3-71-2* | NCO/TEGDMA | .15% | 1% | .50% MDEA | 74 % | |
| Standard-E | NOC/TEGDMA | .15% | - | .80% 4-EDAB | 69 % | |
| SV3-44-2* | NCO/TEGDMA | .15% | 1% | .80% 4-EDAB | 78 % | |
| SV3-44-1* | NCO/TEGDMA | - | 1% | .80% 4-EDAB | 77 % | No CQ |
| SV3-48-4* | NCO/TEGDMA | .15% | 1% | - | 75 % | No amine |
| Standard-Q | NCO/TEGDMA | .15% | - | .80% Qu DMB | 68 % | Diff.amine |
| SV3-79-1* | NCO/TEGDMA | .15% | 1% | .80% Qu DMB | 77 % | Diff.amine |
| Effect of Varying Concentration of Lucerin - No CQ, No Amine | | | | | | |
| SV3-61-4* | NCO/TEGDMA | - | .25% | - | 65 | |
| SV3-61-3* | NCO/TEGDMA | - | .50% | - | 70 | |
| SV3-65-2* | NCO/TEGDMA | - | 1% | - | 72 | |
| SV3-49-4* | NCO/TEGDMA | - | 2% | - | 78 | Yellow color |

*Formulation with Lucerin 8728

Table II illustrates the relative effectiveness of the initiator system when used with a filled resin. FUL-FIL® a product of L.D. Caulk, a division of Dentsply International corresponds to standard-M resin from Table I having 77% milled Raysorb filler (Barium boron aluminum silicate glass). Each of the composites in Table II are made using the resin with the similar identification number in Table I. The percentages are percentage by weight, including the weight of the filler. Data from Table II illustrates that a composite resin with an initiator system which employs camphorquinone, Lucerin 8728 and an amine has a significantly greater depth of cure (DOC) than a corresponding composite that does not use the initiator system of the invention. See for example the properties formulation SV3-71-2, SV3-44-2C, and SV3-79-1C as compared with FUL-FIL® composite, Standard E composite and SV3-46-3C (or Standard Q composite) respectively.

The depth of cure (DOC) was determined by irradiating composite material in rectangular molds 2.5 x 3.5 x 10 mm) for 40 seconds with a PRISMETICS® Light. DOC gives the length of cured material in mm which remains after the uncured portion had been removed with a plastic spatula.

TABLE II

| Formulation of Composites and DOC Values | | | | | | | |
|---|---|---|---|---|---|---|---|
| Formulation | Resin | Milled Raysorb | CQ | Lucerin 8728 | Amine | Additive | DOC** mm |
| | | | in composite | | | | |
| Ful-Fil | 23 % Standard-M | 77 % | .04% | - | .13 % MDEA | - | 5.6 |
| SV3-71-2* | 25 % SV3-71-2 | 75 % | .04% | .25 % | .13 % MDEA | - | 6.1 |
| Standard-EC | 25 % Standard-E | 75 % | .04% | - | .20 % 4-EDAB | - | 6.3 |
| SV3-44-2C* | 25 % SV3-44-2 | 75 % | .04% | .25 % | .20 % 4-EDAB | - | - |
| SV3-44-1C* | 25 % SV3-44-1 | 75 % | - | .25 % | .20 % 4-EDAB | - | 3.4 |
| SV3-48-4C* | 25 % SV3-48-4 | 75 % | .04% | .25 % | - | - | 4.0 |
| SV3-46-3C | 25 % Standard-Q | 75 % | .04% | - | .20 % Qu DMB | - | 5.0 |
| SV3-79-1C* | 25 % SV3-79-1 | 75 % | .04% | .25 % | .20 % Qu DMB | - | 6.3 |
| SV3-65-2C* | 25 % SV3-65-2 | 75 % | - | .25 % | - | - | 4.3 |

*Formulations with Lucerin 8728
**40s Irradiation with Prismetics

Table III illustrates that the maximum Knoop hardness of composites using the camphorquinone,

Lucerin 8728 and amine initiator are greater (all above 58) than similar composites having other initiator systems. Table III also demonstrates that Knoop hardness is measureable at greater depths in said compositions.

The Knoop hardness profiles were obtained by measuring the Knoop Hardness with a Kentron Micro Hardness Tester every 0.5 mm along the surface of specimens which were cured in rectangular molds as described above. The SV3-44-2C composite, for example demonstrates a Knoop hardness of 13 at 5.0 mm.

It should be noted that Uvinol is a U.V. screen that is used to stabilize the composite against discoloration caused by U.V. radiation.

With reference now to FIG. 1, a graphic illustration comparing the hardness at various depths of FUL-FIL® composite (line B, the dashed line) and (line A, the solid line) the composite SV3-71-2C from Table III (FUL-FIL® composite with Lucerin added), is provided. In the illustration KHN is the Knoop hardness, plotted on the Y axis of the graph, and the X axis represents the depth in mm at which the hardness measurement was taken. Each composite was irradiated for 40 seconds under a PRISMETICS® Light apparatus.

Referring now to FIG. 2, a graphic illustration comparing composite SV3-46-3C (line B1, the dashed line) and the composite SV3-79-IC (line A1, the solid line) which is SV3-46-3C with Lucerin added, is provided. The parameters are the same as those described above. Both FIG. 1 and FIG. 2 graphically illustrate that the addition of Lucerin to a composite improves the hardness and the depth of the hardness obtained in a composite material.

TABLE III

| Knoop Hardness as a Function of Depth after 40s Irradiation With PRISMETICS Light | | | | | | | | | | | | |
|---|---|---|---|---|---|---|---|---|---|---|---|---|
| mm Depth | 0.5 | 1.0 | 1.5 | 2.0 | 2.5 | 3.0 | 3.5 | 4.0 | 4.5 | 5.0 | DOC mm | KHNmax |
| FUL-FIL | 43 | 38 | 35 | 30 | 21 | 15 | - | - | - | - | 5.6 | 42.6 |
| SV3-71-2C* | 59 | 58 | 55 | 45 | 34 | 23 | 15 | | | | 6.1 | 58.7 |
| Standard EC | 45 | 43 | 36 | 33 | 27 | 20 | 14 | | | | 6.3 | 45.2 |
| SV3-44-2C** | 58 | 53 | 50 | 45 | 42 | 37 | 33 | 25 | 18 | 13 | 7.0 | 58.0 |
| SV3-46-3C | 47 | 45 | 41 | 35 | 25 | 20 | 13 | - | - | - | 5.0 | 46.7 |
| SV3-79-1C* | 58 | 57 | 53 | 48 | 41 | 32 | 25 | 21 | 14 | - | 6.3 | 58.4 |
| Composite without CQ | | | | | | | | | | | | |
| SV3-82-4C*** | 55 | 52 | 47 | 39 | 31 | 29 | 21 | 15 | - | - | 5.8 | 55.2 |
| Composite without Amine/CQ | | | | | | | | | | | | |
| SV3-65-2C* | 55 | 53 | 52 | 43 | 24 | 16 | - | - | - | - | 4.3 | 54.7 |

*Formulation with Lucerin 8728
**Same as SV3-44-2C except containing 0.38% Uvinol (UV screen)
***Same as SV3-44-2C except containing 0.38% Uvinol (UV screen) and no CQ

Table IV illustrates the effectiveness of cure at various distances from the light source.

TABLE IV

| Conversion in Different Distances from the Light Source | | | |
|---|---|---|---|
| Average distance from the light source in mm | | | |
| DISTANCE | 1.1 ±.07 mm | 2.3 ±.08 mm | 3.5 ±.07 mm |
| Standard EC % conversion (20 second irradiation with PRISMETICSÅ Light) | 74 | 68 | 62 |
| SV3-44-2C % conversion (20 seconds irradiation with PRISMETICSÅ Light) | 78 | 73 | 67 |

Table V illustrates that hardness as measured using a Barcol Medium Hardness tester at the surface T

of the composite was the same (90) whether Lucerin 8728 was used in the composition or not. To approximate the depth of cure, the specimen is removed from a cylinderical mold of 8 mm x 12 mm. The soft, uncured composite material is removed from the underside of the cylinder and the hardness of the bottom surface measured. Uncured, soft material is further removed from the underside until a hardness value of 70 is obtained, which approximates acceptable curing. To achieve a hardness of 70, only 7.7 mm of material remained for FUL-FIL® composite as compared to 8.3 with the material containing 1% Lucerin in FUL-FIL® composite.

The prophy abrasion properties, the transverse strength (in mega pascals), the flexural strength and shrinkage are properties determined as described in copending U.S. SN 040,636 having the same assignee.

TABLE V

**Physical properties of composite hand mixes that contain 1% Lucerin in resin. Two hand mixes were made; one as a control (normal FUL-FIL formulation) the other as a test composite. Both hand mixes contained MDEA as the amine accelerator.**
Properties tested are listed below with results.

| Composite | Physical Properties | | | | | | | |
|---|---|---|---|---|---|---|---|---|
| | Prophy Abrasion ($x10^4$ ml) | | Barcol Hardness | | | Transverse Strength (MPa) | Flexural Modulus (MPa) | Shrinkage (Vol %) |
| | | | T | B | Depth(mm) | | | |
| *1% LR in FUL-FIL | 21.467 | SD 1.86 | 90 | 70 | 8.3 | 146.98 ± 17.90 | 10,368 ±363 | 4.63 ± .16 |
| *STD FUL-FIL | 23.44 | SD 1.65 | 90 | 70 | 7.7 | 136.28 ± 16.26 | 8674 ±309 | 4.80 ± .42 |

As results indicate there is a definite increase in strength in the LR material. This did not show a significant difference in shrinkage or prophy.

Table VI illustrates the properties of a 50:50 BIM/TNCO posterior composite resin as illustrated in copending application SN 040,636. Tests for properties of the resin were carried out using the methods described in SN 040,636. Table VII illustrates properties of composites made using resin compositions described in Table VI. The composites of Table VII comprised 85% filler. The diametral tensile strength, transverse strength and flexural modulus of compositions containing Lucerin 8728 was improved in almost every case over the properties of similar compositions without Lucerin 8728, with best results, in general, being obtained by compositions which contain Lucerin 8728, camphorquinone and an amine.

The Diametral Tensile strength (PSI) and color stability were determined as is known to those skilled in the art.

Cures were obtained using both PRISMA® and PRISMETICS® Lights, both products of L.D. Caulk, a division of Dentsply International Inc.

TABLE VI

| 50/50 BIM/TNCO Posterior Composite Resins With Lucerin | | | | | | | |
|---|---|---|---|---|---|---|---|
| Description | Depth of Cure | | Diametral Tensiles (PSI) | Transverse Strength (MPa) | Flexural Modulus (MPa) | Color Stability | |
| | PRISMA Light | PRISMETICS Light | | | | 24 hr. UV | 7 Days Wet 50 °C |
| CQ .15% MDEA .5% | 2.7 mm | 3.3 mm | 5,761 (361) 5 | 116 (8.6) 6 | 6,950 (622 | Pass | Pass |
| Lucerin 8728 1% (No amine, No CQ) | 1.7 mm | 2.4 mm | 6,489 (527) 6 | 129 (12) 6 | 14,749 (839) 6 | Fail | Pass |
| Lucerin 8728 1% CQ .15% (No amine) | 1.9 mm | 2.5 mm | 6,227 (586) 6 | 147.0 (15) 6 | 14,349 (793) 6 | Fail | Pass |
| CQ .15% Lucerin 8728 1% MDEA 0.5% | 3.0 mm | 3.7 mm | 7,024 (568) 6 | 149 (5.5) 5 | 12,776 (383) 6 | Fail | Pass |
| CQ .15% EDAB 0.65% Uvinol M-40 1.5% | 4.6 mm | 5.5 mm | 7,161 (308) 6 | 145 (3.4) 5 | 10,069 (356) 6 | Pass | Pass |
| CQ .15% EDAB 0.65% Uvinol M-40 1.5% Lucerin 1% | - | 5.6 mm | 6,853 (703) 7 | 147.3 (13.6) 6 | 13,623 (544) 6 | Pass | Pass |

TABLE VII

| 50/50 BIM/TNCO Posterior Composite Resins With Lucerin | | |
|---|---|---|
| Description | Transverse Strength (MPa) | Flexural Modulus (MPa) |
| CQ 0.15%<br>MDEA 0.5% | 53.1 (4.9) 6 | 827 (84) 6 |
| CQ 0.15%<br>EDAB 0.65%<br>Uvinol M-40 1.5% | 88.5 (7.9) 6 | 1,857 (72) 6 |
| Lucerin 1% | 91.0 (8.3) 5 | 2,053 (125) 6 |
| CQ 0.15%<br>MDEA 0.5%<br>Lucerin 1% | 101.2 (4.8) 6 | 2,169 (50) 6 |
| CQ 0.15%<br>EDAB 0.65%<br>Uvinol M-40<br>Lucerin 1% | 94.6 (7.4) 5 | 2,287 (35) 6 |

Referring now to Table VIII and FIG. 3, the environmental resistance of resins and composites to solvents of various solubility parameters was determined using a method described by Wu et al.

Test data was obtained, unless otherwise specifically described, as illustrated by McKinney et al in the Journal of Dental Research 64(11) pp 1326-1331, November 1985.

Resin or composite disks of approximately 8 mm in diameter and 1 mm high, were irradiated between glass slides for 1 minute from each side and stored in a dry oven at 37°.

After 24 hours the specimens were measured for their initial Knoop Hardness (8 indentations per specimen).

Two specimens each were immersed in the following solvents: heptane, 100% ethanol, mixtures of 75, 50, 25% ethanol in water, and distilled water. The solubility parameters for the solvents range from 1.51 x $10^{-4}$ for heptane to 4.8 x $10^{-4}$ $J^{1/2}m^{-3/2}$ for pure water.

After 3 weeks final Knoop hardness was measured and expressed in % of the initial hardness. In FIG. 3, the Oral Environmnent Resistance Index (OER) is the area under the hardness retention curve and provides a numerical value for a given material for its resistance to damage in organic fluids.

The data obtained from the above described tests are compiled in Table VIII. The values given in the Table are the percent remaining hardness after the test for four specimens of FUL-FIL® composite and four specimens of composite SV3-71-2C. The percent average remaining hardness for the four samples of FUL-FIL® composite in the 6 solvents tested was 61.45% (OER). The percent average remaining hardness for the four samples of SV3-71-2C in the 6 solvents tested was 73.46% (OER), illustrating that a composite formulation including Lucerin has improved resistance in the oral environment.

The data of the Table is illustrated in FIG. 3. In FIG. 3, FUL-FIL® composite is represented by B3 (the dashed line), and SV3-71-2C composite is represented by A3 (the solid line). The points plotted in the graph represent the average of the four samples of each composite.

TABLE VIII

| % REMAINING HARDNESS | | | | | | |
|---|---|---|---|---|---|---|
| Composite FUL-FIL | Heptane | EtOH 100% | EtOH 75% | EtOH 50% | EtOH 25% | Water |
| 1 | 89 * | 47 | 49 | 52 | 57 | 89 |
| 2 | 90 | 51 | 50 | 51 | 62 | 87 |
| 3 | 87 | 50 | 50 | 50 | 62 | 90 |
| 4 | 85 | 52 | 53 | 53 | 61 | 83 |
| SV3-71-2C | | | | | | |
| 1 | 98 | 64 | 59 | 67 | 73 | 81 |
| 2 | 101 | 65 | 63 | 68 | 71 | 85 |
| 3 | 99 | 67 | 62 | 68 | 73 | 83 |
| 4 | 96 | 66 | 63 | 70 | 73 | 82 |

* Each number represents the average of four measurements

While present embodiments of the invention and methods practicing the same have been illustrated and described, it will be recognized by those skilled in the art that this invention may be otherwise variously embodied and practiced within the scope of the following claims.

## Claims

1. A photopolymerizable composition for dental use comprising at least one olefinically unsaturated monomer and a catalyst comprising acyl phosphonate as a photoinitiator/photoaccelerator, said composition being curable in the visible light range of the spectrum.

2. The composition of Claim 1 wherein the monomer includes at least one acrylic or methacrylic ester.

3. The composition of Claim 1 which includes a vicinal diketone.

4. The composition of Claim 3 which includes an amine.

5. The composition of Claim 1 which includes a tertiary amine.

6. The composition of Claim 1 wherein the composition contains a finely divided inorganic filler.

7. The composition of Claim 1 wherein said acylphosphonate has the formula

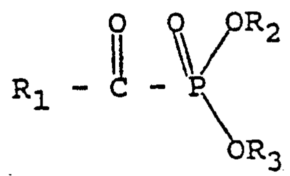

wherein $R_1$ is aryl, alkyl, OH or OR where R is aryl or alkyl;
$R_2$ and $R_3$ may be the same or different and together may form a ring and are selected from the group comprising aryl, alkyl and H;
wherein alkyl is a saturated or unsaturated chain comprising from 1 to 18 carbon atoms, and aryl represents an aromatic carbon or heterocyclic ring or rings having 4-10 carbon atoms which may have substituents comprising halogen and lower alkyl totaling from 1-6 additional carbon atoms.

8. The composition of Claim 7 wherein said alkyl contains a methacrylate or acrylate moiety.

9. The composition of Claim 1 wherein said catalyst comprises benzoyl di (2,6 dimethylphenyl) phosphonate.

10. The use of the composition of Claim 1 for treating teeth by its application to a prepared tooth.

11. A photopolymerizable composition comprising at least one olefinically unsaturated monomer and a catalyst system comprising acyl phosphonate.

12 The composition of Claim 11 which includes a vicinal diketone.

13. The composition of Claim 12 which includes an amine.

14. The use of the composition of Claim 12 for treating teeth by its application to a prepared tooth.

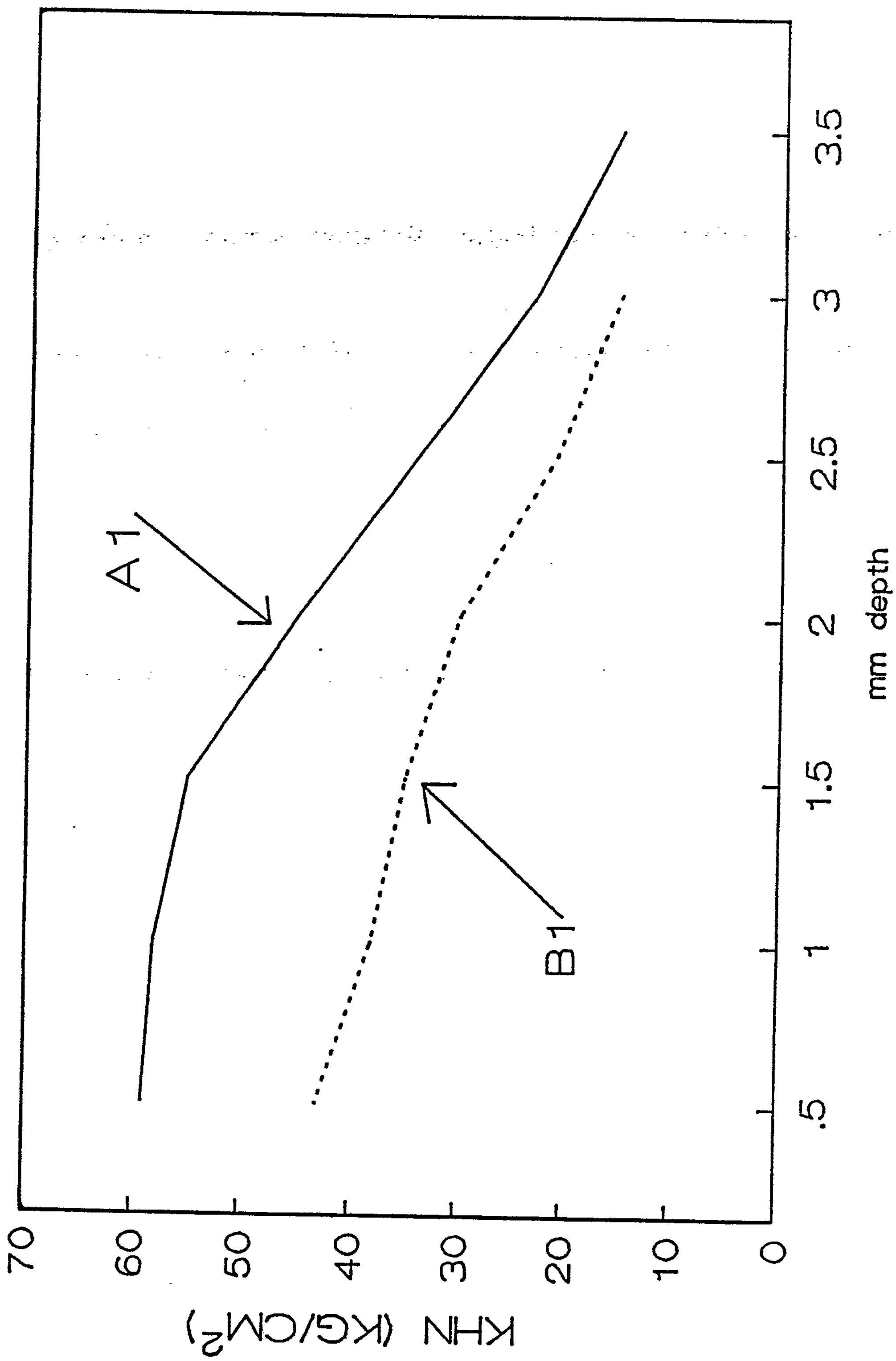


FIG. 1

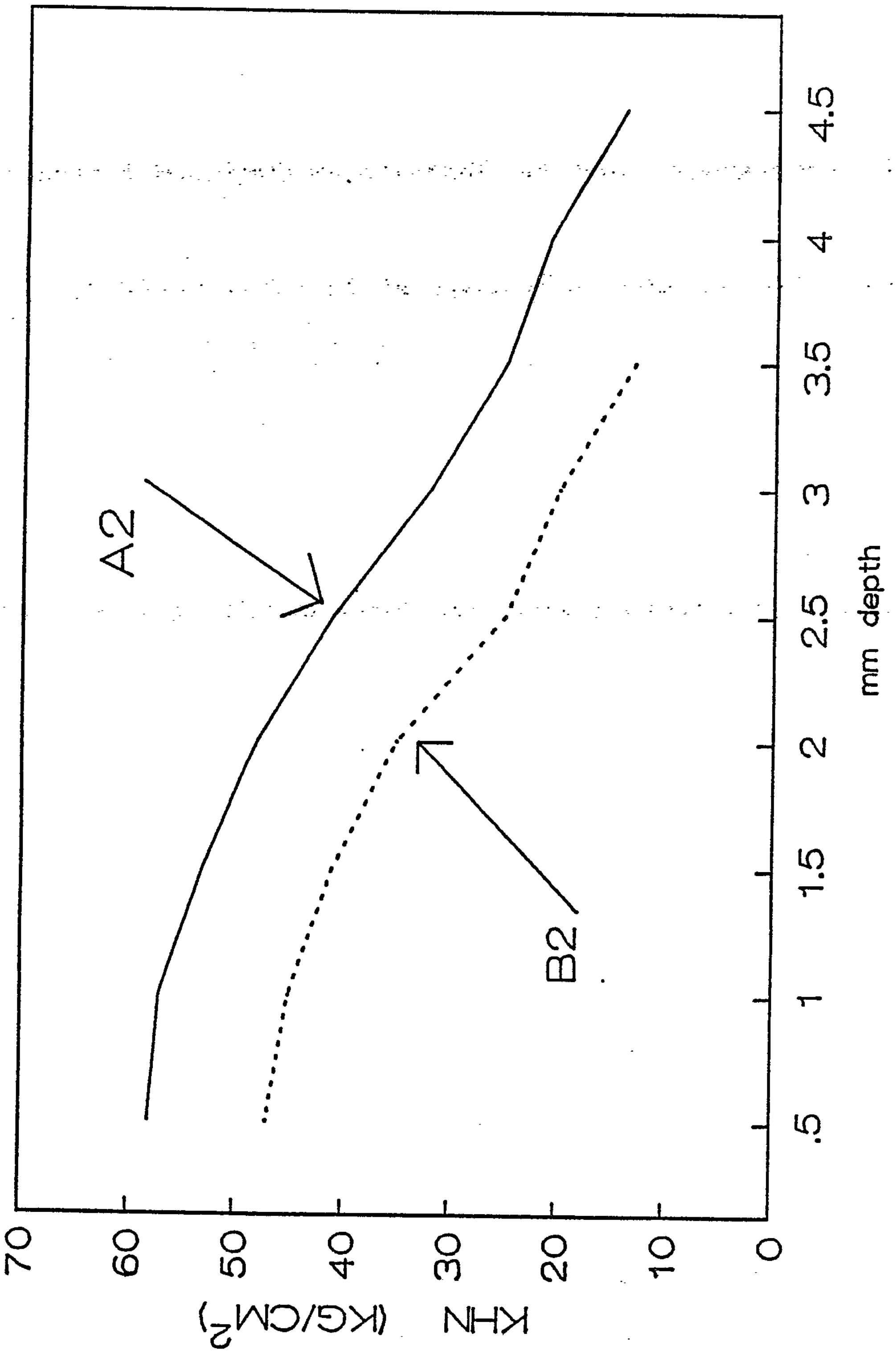


FIG. 2

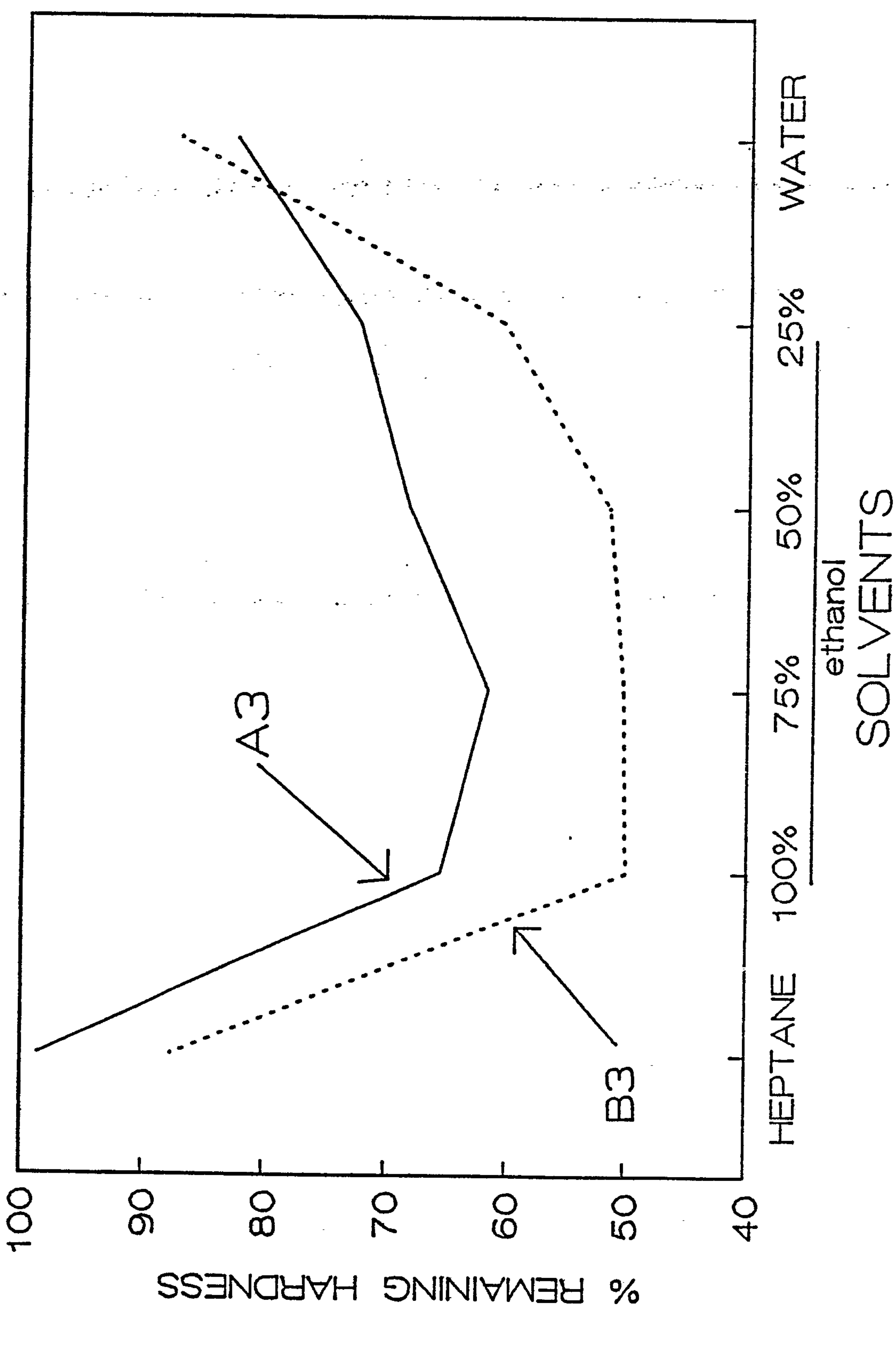
A3
B3
100
90
80
70
60
50
40
% REMAINING HARDNESS
HEPTANE
100%
75%
50%
25%
WATER
ethanol
SOLVENTS

FIG. 3